# EUROPEAN PATENT SPECIFICATION

(11) **EP 1 793 853 B1**
(45) Date of publication and mention of the grant of the patent: **21.01.2009**
(21) Application number: 05784220.5
(22) Date of filing: 22.09.2005
(51) Int. Cl.: A61K 39/35, A61K 39/39

(54) **LIQUID ALLERGY VACCINE FORMULATION FOR OROMUCOSAL ADMINISTRATION**
FLÜSSIGE ALLERGIE-IMPFSTOFFFORMULIERUNG ZUR OROMUKOSALEN VERABREICHUNG
FORMULATION DE VACCIN LIQUIDE CONTRE UNE ALLERGIE POUR UNE ADMINISTRATION SUR LES MUQUEUSES BUCCALES

(30) Priority: 27.09.2004 DK 200401468; 27.09.2004 US 613892 P
(43) Date of publication of application: 13.06.2007
(73) Proprietor: Alk-Abelló A/S, 2970 Hørsholm (DK)
(72) Inventor: BRIMNES, Jens, 2730 Herlev (DK); KILDSGAARD, Jens, 2840 Holte (DK)
(74) Representative: Benthin, Stig
(86) International application number: PCT/DK2005/000601
(87) International publication number: WO 2006/034707

(56) References cited:
- WO-A-00/45847
- WO-A-02/40676
- WO-A-03/096869
- WO-A-20/04047794
- US-A- 3 148 121
- US-A1- 2004 013 695
- FANTA C ET AL: "SYSTEMIC IMMUNOLOGICAL CHANGES INDUCED BY ADMINISTRATION OF GRASS POLLEN ALLERGENS VIA THE ORAL MUCOSA DURING SUBLINGUAL IMMUNOTHERAPY" INTERNATIONAL ARCHIVES OF ALLERGY AND IMMUNOLOGY, vol. 120, no. 3, November 1999 (1999-11), pages 218-224, XP008044918 ISSN: 1018-2438
- WALKER SAMANTHA M ET AL: "Grass pollen immunotherapy for seasonal rhinitis and asthma: A randomized, controlled trial" JOURNAL OF ALLERGY AND CLINICAL IMMUNOLOGY, vol. 107, no. 1, January 2001 (2001-01), pages 87-93, XP002330540 ISSN: 0091-6749

## Description

### TECHNICAL FIELD

The present invention relates to an allergy vaccine formulation for oromucosal administration.

### BACKGROUND OF THE INVENTION

Allergy is a major health problem in countries where Western lifestyle is adapted. Furthermore, the prevalence of allergic disease is increasing in these countries. Although allergy in general may not be considered a life-threatening disease, asthma annually causes a significant number of deaths. An exceptional prevalence of about 30% in teenagers conveys a substantial loss in quality of life, working days and money, and warrants a classification among major health problems in the Western world.

Allergy is a complex disease. Many factors contribute to the sensitisation event. Among these is the susceptibility of the individual defined by an as yet insufficiently understood interplay between several genes. Another important factor is allergen exposure above certain thresholds. Several environmental factors may be important in the sensitisation process including pollution, childhood infections, parasite infections, intestinal microorganisms, etc. Once an individual is sensitised and the allergic immune response established, the presence of only minute amounts of allergen is efficiently translated into symptoms.

The natural course of allergic disease is usually accompanied by aggravation at two levels. Firstly, a progression of symptoms and disease severity, as well as disease progression, for example from hay fever to asthma.

Secondly, dissemination in offending allergens most often occurs resulting in allergic multi-reactivity. Chronic inflammation leads to a general weakening of the mucosal defense mechanisms resulting in unspecific irritation and eventually destruction of the mucosal tissue. Infants may become sensitised primarily to foods, i.e. milk, resulting in eczema or gastrointestinal disorders; however, most often they outgrow these symptoms spontaneously. These infants are at risk of developing inhalation allergy later in their lives.

The most important allergen sources are found among the most prevalent particles of a certain size in the air we breathe. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis yet other pollens may dominate, such as birch pollen in Northern and Central Europe, ragweed in the Eastern and Central United States, and Japanese cedar pollen in Japan. Insects, i.e. bee and wasp venoms, and foods each account for approximately 2% of all allergies.

Allergy, i.e. type I hyper-sensitivity, is caused by an inappropriate immunological reaction to foreign non-pathogenic substances. Important clinical manifestations of allergy include asthma, hay fever, eczema, and gastro intestinal disorders. The allergic reaction is prompt and peaks within 20 minutes upon contact with the offending allergen. Furthermore, the allergic reaction is specific in the sense that a particular individual is sensitised to particular allergen(s), whereas the individual does not necessarily show an allergic reaction to other substances known to cause allergic disease. The allergic phenotype is characterized by a pronounced inflammation of the mucosa of the target organ and by the presence of allergen specific antibody of the IgE class in the circulation and on the surfaced of mast-cells and basophils.

An allergic attack is initiated by the reaction of the foreign allergen with allergen specific IgE antibodies, when the antibodies are bound to high affinity IgE specific receptors on the surface of mast-cells and basophils. The mast-cells and basophils contain preformed mediators, i.e. histamine, tryptase, and other substances, which are released upon cross-linking of two or more receptor-bound IgE antibodies. IgE antibodies are cross-linked by the simultaneous binding of one allergen molecule. It therefore follows that a foreign substance having only one antibody binding epitope does not initiate an allergic reaction. The cross-linking of receptor bound IgE on the surface of mast-cells also leads to release of signaling molecules responsible for the attraction of eosinophils, allergen specific T-cells, and other types of cells to the site of the allergic response. These cells in interplay with allergen, IgE and effector cells, lead to a renewed flash of symptoms occurring 12-24 hours after allergen encounter (late phase reaction).

Allergy disease management comprises diagnosis and treatment including prophylactic treatments. Diagnosis of allergy is concerned with by the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick test, blood test, or provocation test.

The therapeutic options fall in three major categories. The first opportunity is allergen avoidance or reduction of the exposure. Whereas allergen avoidance is obvious e.g. in the case of food allergens, it may be difficult or expensive, as for house dust mite allergens, or it may be impossible, as for pollen allergens. The second and most widely used therapeutic option is the prescription of classical symptomatic drugs like anti-histamines and steroids. Symptomatic drugs are safe and efficient; however, they do not alter the natural cause of the disease, neither do they control the disease dissemination. The third therapeutic alternative is specific allergy vaccination that in most cases reduces or alleviates the allergic symptoms caused by the allergen in question.

Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and in addition, most patients experience a relief in disease severity and symptoms experienced, or at least an arrest in disease aggravation. Thus, specific allergy vaccination has preventive effects reducing the risk of hay fever developing into asthma, and reducing the risk of developing new sensitivities.

The immunological mechanism underlying successful allergy vaccination is not known in detail. A specific immune response, such as the production of antibodies against a particular pathogen, is known as an adaptive immune response. This response can be distinguished from the innate immune response, which is an unspecific reaction towards pathogens. An allergy vaccine is bound to address the adaptive immune response, which includes cells and molecules with antigen specificity, such as T-cells and the antibody producing B-cells. B-cells cannot mature into antibody producing cells without help from T-cells of the corresponding specificity. T-cells that participate in the stimulation of allergic immune responses are primarily of the Th2 type. Establishment of a new balance between Th1 and Th2 cells has been proposed to be beneficial and central to the immunological mechanism of specific allergy vaccination. Whether this is brought about by a reduction in Th2 cells, a shift from Th2 to Th1 cells, or an up-regulation of Th1 cells is controversial. Recently, regulatory T-cells have been proposed to be important for the mechanism of allergy vaccination. According to this model regulatory T-cells, i.e. Th3 or Tr1 cells, down-regulate both Th1 and Th2 cells of the corresponding antigen specificity. In spite of these ambiguities it is generally believed that an active vaccine must have the capacity to stimulate allergen specific T-cells, preferably TH1 cells.

Specific allergy vaccination is, in spite of its virtues, not in widespread use, primarily for two reasons. One reason is the inconveniences associated with the traditional vaccination programme that comprises repeated vaccinations i.a. injections over a several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing.

Conventional specific allergy vaccination is therefore carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment. There is no doubt that a vaccine based on a different route of administration would eliminate or reduce the risk for allergic side reactions inherent in the current subcutaneous based vaccine as well as would facilitate a more widespread use, possibly even enabling self vaccination at home.

Attempts to improve vaccines for specific allergy vaccination have been performed for over 30 years and include multifarious approaches. Several approaches have addressed the allergen itself through modification of the IgE reactivity. Others have addressed the route of administration.

The immune system is accessible through the oral cavity and sublingual administration of allergens is a known route of administration. Administration may be carried out by placing the vaccine formulation under the tongue and allowing it to remain there for a short period of time, e.g. 30 to 60 seconds.

Conventionally allergy vaccine using the oromucosal route consists of the up to daily dosing of a solution of the allergen. In comparison, the therapeutic (accumulated) maintenance doses given exceeded the maintenance of the comparable subcutaneous dose by a factor 5-500.

Commercial allergy vaccines for administration via subcutaneous injection comprising an allergen and aluminium hydroxide functioning as an adjuvant are known. Also, the use of oxygen-containing metal salts in vaccines for delivery via the mucosa of the gastrointestinal tract has been proposed. In the following a discussion of the properties of oxygen-containing metal salts and various hypotheses on how oxygen-containing metal salts functions in injection vaccines and in vaccines for delivery via the mucosa of the gastrointestinal tract are given.

Oxygen-containing metal salts can be characterised by a variety of physical-chemical parameters like adsorption, solubility and dissolution properties, ionic charge measured as the isoelectric point pl (pH where the net charge of the substance is zero for a dissociationable compound), dissociation constants, complex coordination, electronic configurations, valence, bonding orbitals and antibonding orbitals, depot properties, adhesion properties, surface characteristics, particle characteristics, and adjuvanticity.

It is believed that the biologically active substance is adsorbed (or coupled) to the oxygen-containing metal salt, and this adsorption contributes to the efficacy of the vaccine. Several factors may be important or influence the adsorption between the active substance and the oxygen-containing metal salt (see e. g. P. M. Callahan et al., Pharmaceutical Research Vol. 8, No. 7,851-858 (1991), and Vaccine Design. The Subunit and Adjuvant Approach). These factors include pH, the length of time the adsorption reaction is carried out for, mixing conditions, concentrations of the various components in the vaccines, containers, temperature, storage, buffer and excipients. It has further been found that the adsorption of the active substance may be influenced by the net/overall charge of the metal salt and the charge of the active substance, both of which are pH dependent. A further feature believed to be of importance is the solubility of the oxygen-containing metal salts.

The oxygen-containing metal salt may further have a depot effect. A depot effect means that the active substance will be released gradually from the vaccine. The active substance will thus be retained with the oxygen-containing metal salt (s) and released gradually therefrom. This is believed to have a number of beneficial effects, e. g. prolonged stimulation, beneficial drug release, and protection of the biological interactive substances against environmental conditions. It is further believed that the oxygen-containing metal salt may possess certain entrapment properties, thus retaining the active substance to be delivered.

Another feature of oxygen-containing salts is the protection of the active substance either by maintaining the ideal pH for the active substance in the microenvironment, thus preventing acid degradation, or by protecting the active substance against enzymatic degradation thereby allowing the substance to be delivered.

Furthermore, some of the oxygen-containing metal salts have a buffer capacity. This may result in an in vivo microenvironment within the vaccine formulation, which protects the active substance from the degradable environment. This may e. g. be an advantage in the stomach or intestine where there is a risk of acid and enzymatic degradation, respectively.

A further feature of the oxygen-containing metal salt (s) is their capability to adhere to the mucosal membrane, or the effect they may exert on the gastrointestinal movement, e. g. slowing it down. The extended time of passage through the intestine may be beneficial due to the enhanced possibility of prolonging the time allowed for interaction of the active substance with the target tissue (mucosal membrane), thereby leading to increased transport of the active substances via the mucosal membrane.

It is further believed that the oxygen-containing metal salt (s) can be designed to have specific preference for specific mucosal tissues e. g. GALT and Peyers patch, further enhancing the delivery of the active substances at a relevant target site (mucosal tissue). For several of the oxygen-containing metal salts (e. g. Al(OH)₃, AlPO₄, Ca₃PO₄) the particle size range is between 0.5 and 15 µm.

WO 00/45847 relates to an allergy vaccine for mucosal administration containing an allergen and an oxygen-containing metal salt. The Examples describe liquid allergy vaccines for peroral and intraperitoneal administration, i.e. delivery via the gastrointestinal tract, comprising aluminium hydroxide and an allergen in the form of an extract of the grass Phleum pratense. In the general part of the description it is speculated that the oxygen-containing metal salt increases the immune stimulating effect via one or more of several factors including a protective effect against degradation in the gastrointestinal environment of the allergen protein, mucosa-adhesive properties to increase the residence time in the gastrointestinal tract, a depot effect, and a particle size suitable for uptake via GALT or Peyers patches.

WO 04/047794 discloses a solid fast-dispersing dosage form for sublingual administration of an allergy vaccine.

One commercial allergy vaccine for sublingual administration comprises an allergen dissolved in a liquid mixture of 50 % glycerol and 50 % water.

The object of the present invention is to provide an improved liquid allergy vaccine for oromucosal, in particular sublingual, administration.

### SUMMARY OF THE INVENTION

This object is obtained by the present invention, which relates to the use of a composition comprising an allergen and an adjuvant selected from the group consisting of oxygen-containing metal salts for the manufacture of a liquid formulation for preventing or treating allergy in a subject by oromucosal administration.

It has surprisingly been shown that for oromucosal administration a vaccine formulated as a liquid and containing an oxygen-containing metal salt is particularly suitable. In particular such a vaccine formulation has a surprisingly improved effect with respect to the ability to activate the immune system. Such improved properties could not be anticipated, since the functional properties of the oxygen-containing metal salt, which are believed to be responsible for the improved properties of an allergy vaccine for delivery via the mucosa of the gastro-intestinal tract and of an injection vaccine, are not relevant for an allergy vaccine intended for sublingual administration, wherein the vaccine is contacted with the sublingual mucosa for a short pre-determined period of time.

A further advantage of the use of the invention is that the oxygen-containing metal salt imparts a gel-like structure on the liquid vaccine formulation thereby making it easier to keep in place in the mouth, in particular under the tongue, for the required period of time, and hence it facilitates treatment protocol compliance and correct dosing.

Yet a further advantage of the use of the invention is that the mucosal antibody response is believed to be stronger in the mucosal compartment, wherein the stimulation occurs, than in other mucosal compartments. Therefore, oromucosal administration is believed to elicit the strongest antibody response in the mucosa of the oral cavity or pharynx, with which the allergens comes into contact and hence result in the most efficient treatment of allergy.

### DETAILED DESCRIPTION OF THE INVENTION

### Allergen

The allergen of the formulation according to the present invention may be any naturally occurring protein that has been reported to induce allergic, i.e. IgE mediated, reactions upon their repeated exposure to an individual. Examples of naturally occurring allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens. Important pollen allergens from trees, grasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria . Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are i.a. such originating from the genera Alternaria and Cladosporium.

In a particular embodiment of the invention the allergen is Bet v 1, Aln g 1, Cor a 1 and Car b 1, Que a 1, Cry j 1, Cry j 2 , Cup a 1, Cup s 1, Jun a 1, Jun a 2, jun a 3, Ole e 1 , Lig v 1, Pla I 1, Pla a 2, Amb a 1, Amb a 2, Amb t 5, Art v 1, Art v 2 Par j 1 , Par j 2, Par j 3, Sal k 1, Ave e 1, Cyn d 1, Cyn d 7, Dac g 1, Fes p 1, Hol l 1, Lol p 1 and 5, Pha a 1, Pas n 1, Phl p 1, Phl p 5, Phl p 6, Poa p 1, Poa p 5, Sec c 1, Sec c 5, Sor h 1, Der f 1 , Der f 2, Der p 1, Der p 2, , Der p 7, Der m 1, Eur m 2, Gly d 1, Lep d 2, Blo t 1, Tyr p 2, Bla g 1, Bla g 2, Per a 1, Fel d 1, Can f 1, Can f 2 , Bos d 2, Equ c 1, Equ c 2, Equ c 3 , Mus m 1, Rat n 1, Apis m 1, Api m 2 , Ves v 1, Ves v 2, Ves v 5, Dol m 1, Dil m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4, Ara h 5 or shufflant hybrids from Molecular Breeding of any of these.

In a preferred embodiment of the invention the allergen is selected form the group consisting of a grass pollen allergen, a dust mite allergen, a ragweed allergen, a cedar pollen, a cat allergen and a birch allergen.

In yet another embodiment of the invention the formulation comprises at least two different types of allergens either originating from the same allergic source or originating from different allergenic sources e.g. grass group 1 and grass group 5 allergens or mite group 1 and group 2 allergens from different mite and grass species respectively, weed antigens like short and giant ragweed allergens, different fungis allergens like alternaria and cladosporium, tree allergens like birch, hazel, hornbeam, oak and alder allergens, food allergens like peanut, soybean and milk allergens.

The allergen incorporated into the formulation may be in the form of an extract, a purified allergen, a modified allergen, a recombinant allergen or a mutant of a recombinant allergen. An allergenic extract may naturally contain one or more isoforms of the same allergen, whereas a recombinant allergen typically only represents one isoform of an allergen. In a preferred embodiment the allergen is in the form of an extract. In another preferred embodiment the allergen is a recombinant allergen. In a further preferred embodiment the allergen is a naturally occurring low IgE-binding mutant or a recombinant low IgE-binding mutant.

Allergens may be present in equi-molar amounts or the ratio of the allergens present may vary preferably up to 1:20.

In a further embodiment of the invention the low IgE binding allergen is an allergen according to WO 99/47680, WO 02/40676 or WO 03/096869 A2.

Classical incremental dosage desensitisation, where the dose of allergen in the form of a fast dispersing solid dosage form is increased to a certain maximum relieves the symptoms of allergy. The preferred potency of a unit dose of the dosage form is from 150 - 1000000 SQ-u/dosage form, more preferred the potency is from 500 - 500000 SQ-u/dosage form and more preferably the potency is from 1000 - 250000 SQ-u/dosage form, even more preferred 1500-125000 SQ-u/dosage form most preferable 1500-75000 SQ-u/dosage form.

In another embodiment of the invention the dosage form is a repeated mono-dose, preferably within the range of 1500-75000 SQ-u/dosage form.

### Oxygen-containing metal salts

In a preferred embodiment of the invention, the metal cation of the oxygen-containing metal salt is selected from the group consisting of Al, K, Ca, Mg, Zn, Ba, Na, Li, B, Be, Fe, Si, Co, Cu, Ni, Ag, Au and Cr.

The anion of the oxygen-containing compound may be any oxygen-containing anion, including an organic or inorganic anion, or a combination of organic and inorganic anions. Examples of suitable oxygen-containing metal salts are e.g. those, wherein the anion is selected from the group consisting of sulphates, hydroxides, phosphates, nitrates, iodates, bromates, carbonates, hydrates, acetates, citrates, oxalates, and tartrates, as well as mixed forms thereof. The oxygen-containing metal salts further comprise coordination complexes. A definition of coordination complexes is given in e.g. The Handbook of Chemistry and Physics 56 Ed., Section B, Chapter 7 (197576).

Within the present context, the expression "mixed forms" is intended to include combinations of the various anions as well as combinations with e. g. chlorides, and sulphides.

Examples of oxygen-containing metal salts according to the invention are aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, zinc sulphate, and barium sulphate.

Most preferred are aluminium hydroxide, aluminium phosphate, aluminium acetate, calcium phosphate, calcium tartrate and zinc sulphate.

The pI of the oxygen-containing metal salt is typically in the range of 2-11. The pI for allergen proteins is typically in the range of 4-9. Preferably, the allergen and oxygen-containing metal salt are selected so that the pI of the allergen is lower than the pI of the oxygen-containing metal salt.

When using e.g. aluminium hydroxide as oxygen-containing metal salt, the concentration of aluminium hydroxide in the formulation is preferably 0.035-1000 mg/ml, more preferably 0.10-100 mg/ml, more preferably 0.25-10 mg/ml, and most preferably 0.5-5 mg/ml. For the other oxygen-containing metal salts, the concentration of the metal salt is preferably 0.035-1000 mg/ml, more preferably 0.35-100 mg/ml, more preferably 0.7-50 mg/ml, and most preferably 1.0-20 mg/ml. The concentration of allergen in the formulation is preferably 0.01-100 mg/ml, more preferably 0.1-10 mg/ml. The ratio of oxygen-containing metal salt to allergen is preferably from 0.1 to 100, more preferably from 1 to 20. The degree of allergen adsorbed to the oxygen-containing metal salt is typically from 5 to 99 %, more preferably from 10 to 99 % of the added amount. The adsorption of allergen to the oxygen-containing metal salt depends on the buffer system and the reaction conditions, including temperature and reaction time, under which the adsorption takes place.

### Oromucosal administration

The immune system is accessible through the oral cavity and sublingual administration of allergens is a known route of administration. Administration may be carried out by placing the vaccine formulation under the tongue and allowing it to remain there for a short period of time, e.g. 30 to 60 seconds.

Oromucosal administration comprises any administration method, wherein the formulation in part or in full comes into contact with the mucosa of the oral cavity and/or the pharynx of the patient. Oromucosal administration methods include sublingual administration and buccal administration.

In one embodiment of the invention, the subject is subjected to a vaccination protocol comprising daily administration of the vaccine. In another embodiment of the invention the vaccination protocol comprises administration of the vaccine every second day, every third day or every fourth day. For instance, the vaccination protocol comprises administration of the vaccine for a period of more than 4 weeks, preferably more than 8 weeks, more preferably more than 12 weeks, more preferably more than 16 weeks, more preferably more than 20 weeks, more preferably more than 24 weeks, more preferably more than 30 and most preferably more than 36 weeks.

The period of administration may a continuous period. Alternatively, the period of administration is a discontinuous period interrupted by one or more periods of non-administration. Preferably, the (total) period of non-administration is shorter than the (total) period of administration.

In a further embodiment of the invention, the vaccine is administered to the patient once a day. Alternatively, the vaccine is administered to the patient twice a day. The vaccine may be a uni-dose vaccine.

### Liquid vaccine formulation

Aqueous solutions of oxygen-containing metal salts typically have the form of gels. As mentioned above, the concentration of aluminium hydroxide in the formulation is preferably 0.035-1000 mg/ml, more preferably 0.10-100 mg/ml, more preferably 0.25-10 mg/ml, and most preferably 0.5-5 mg/ml. However, the formulation of the invention may also have the form of a highly concentrated gel or gel-like formulation.

In a particular embodiment of the invention the formulation according to the invention further comprises an enhancer. An enhancer is a substance, which increases the bioavailability of a pharmaceutically active substance i) by increasing the penetration of the active substance across the biomembrane by opening the paracellular pathways (tight junctions), i.e. increasing the absorption of the active substance, and/or ii) by the bioadhesive effect of the enhancer, which increases the period of absorption.

Examples of enhancers are an alcohol, e.g. ethanol, chitosan, chitin, propylene glycol, glycerol, dimethyl sulfoxide, dimethyl formamide, dimethyl acetamide, preferably glycerol. The enhancer is preferably present in an amount of from 20 % to 80 % (v/v), more preferably from 30 % (v/v) to 70 % (v/v), and most preferably from 40 % (v/v) to 60 % (v/v).

It is to be understood that the formulation of the invention may further comprise additional adjuvants and other excipients suitable for such type of formulation. Such additional adjuvants and excipients are well-known to the person skilled in the art and include i.a. solvents, emulsifiers, wetting agents, plasticizers, colouring substances, fillers, preservatives, viscosity adjusting agents, buffering agents, mucoadhesive substances, and the like. Examples of formulation strategies are well-known to the person skilled in the art.

The additional adjuvant may be any conventional adjuvant, including heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1β, IL-2, IL-7, IL-12, INFγ), GM-CSF, MDF derivatives, CpG oligonucleotides, LPS, MPL, phosphophazenes, Adju-Phos®, glucan, antigen formulation, liposomes, DDE, DHEA, DMPC, DMPG, DOC/Alum Complex, Freund's incomplete adjuvant, ISCOMs®, LT Oral Adjuvant, muramyl dipeptide, monophosphoryl lipid A, muramyl tripeptide, and phospatidylethanolamine.

The formulation of the present invention may be prepared by dissolving the oxygen-containing metal salt as well as additional adjuvants and other excipients, if any, in a solvent, preferably water, adding the allergen and allowing the allergen and oxygen-containing metal salt to react for a period of time. The reaction period may be from 0.1 to 48 hours, preferably from 12 to 24 hours. The reaction is preferably carried out at a temperature of from 4 to 45 °C, more preferably from 4 to 20 °C.

### DEFINITIONS

The term "oromucosal administration" refers to a route of administration where the dosage form is placed under the tongue or anywhere else in the oral cavity to allow the active ingredient to come in contact with the mucosa of the oral cavity or the pharynx of the patient in order to obtain a local or systemic effect of the active ingredient. An example of an oromucosal administration route is sublingual administration.

The term "sublingual administration" refers to a route of administration, where a dosage form is placed underneath the tongue in order to obtain a local or systemic effect of the active ingredient.

The term "liquid" means an oxygen-containing metal salt dissolved or added to a solvent in any concentration, including solutions and gels with low and high viscosity.

The term "SQ-u" means SQ-Unit: The SQ-Unit is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.

The term "treating" means partly of wholly curing, alleviating symptoms or inhibiting causes of symptoms.

The term "preventing" means type of prophylactic treatment.

### EXAMPLES

### Example 1: Sublingual treatment of mice with a liquid formulation of aluminium hydroxide and the grass allergen Phleum pratense (Phl p)

### Treatment protocol

Mice were subjected to a treatment protocol comprising a sensitisation treatment consisting of three intraperitoneal (i.p.) injections and a SLIT treatment consisting of six weeks of one daily administration five days every week of a liquid formulation having one of the following compositions: 1) 5,000 SQ-u Phl p and no aluminium hydroxide (7 mice), 2) 5,000 SQ-u and aluminium hydroxide (Alhydrogel ® 1.3 %) in a concentration corresponding to 1.25 mg Al/ml (8 mice), 3) 500 SQ-u and aluminium hydroxide (Alhydrogel ® 1.3 %) in a concentration corresponding to 1.25 mg Al/ml (10 mice), and 4) no grass allergen and no aluminium hydroxide (10 mice). For the SLIT treatment each dose consisted of a volume of 5 µl liquid formulation, i.e. each dose contained an amount of 1.25/20 mg Al. For the intraperitoneal injections each dose consisted of a volume of 0.25 ml liquid formulation, i.e. each dose contained an amount of 1.25/4 mg Al. The second i.p. injection was given two week after the first, and the third was given one week after the second. The SLIT treatment was commenced one week after the third i.p. treatment.

The liquid formulation was prepared by adding Phl p to coca buffer and mixing it with aluminium hydroxide. The aluminium hydroxide was diluted by a factor of five from a solution of Alhydrogel ® 1.3 % obtained from Brenntag (content of ash residue (Al₂O₃): 1.3 % w/w; content of corresponding Al(OH)₃: 1.99 % w/w; content of aluminium: 6.25 mg Al/ml ± 5%). Coca buffer contains 0.5 % (w/v) sodium chloride and 0.25 % (w/v) sodium hydrogen carbonate.

At the end of the treatment protocol, broncho-alveolar lavage (BAL) was carried out for each mouse to obtain a sample, and the level of IgA antibody in the BAL was determined using the following assay:

### IgA assay

Estapore magnetic beads (Estapore IB-MR/0,86) coupled to goat a-mouse IgA are incubated with BAL. Then washing and incubation with biotinylated allergen is carried out. Then washing and incubation with streptavidin labeled LITE reagent is carried out, and after washing light luminescence is measured in a luminometer (Magic Lite Analyser EQ).

### Results

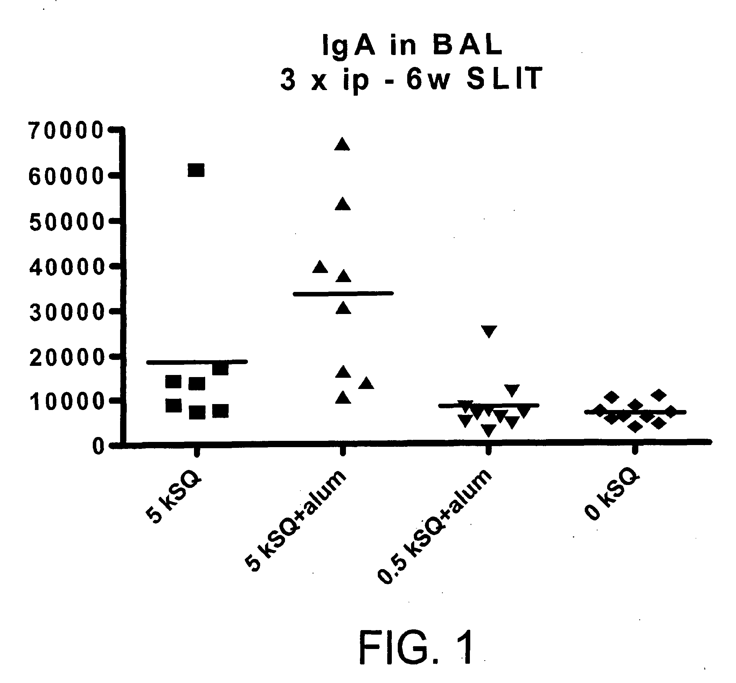

The results are shown in Fig. 1. As will appear from Fig. 1 the use of aluminium hydroxide significantly increases the BAL IgA level for an allergen dose of 5,000 SQ-u.

## Claims

1. Use of a composition comprising an allergen and an adjuvant selected from the group consisting of oxygen-containing metal salts for the manufacture of a liquid formulation for preventing or treating allergy in a subject by oromucosal administration.

2. Use according to claim 1, wherein the oxygen-containing metal salt is selected form the group consisting of aluminium hydroxide, aluminium phosphate and calcium phosphate.

3. Use according to claim 1, wherein the oxygen-containing metal salt is aluminium hydroxide.

4. Use according to claim 3, wherein the concentration of oxygen-containing metal salt is 0.035-1000 mg/ml, more preferably 0.10-100 mg/ml, more preferably 0.25-10 mg/ml, and most preferably 0.5-5 mg/ml.

5. Use according to any of claim 1-4, wherein the concentration of allergen is 0.01-100 mg/ml, more preferably 0.1-10 mg/ml.

6. Use according to any of the preceding claims, wherein the allergen is selected form the group consisting of a grass pollen allergen, a dust mite allergen, a ragweed allergen, a cedar pollen or a cat allergen and a birch allergen.

7. Use according to any of the preceding claims, wherein the oromucosal administration is sublingual administration.

8. Use according to any of the preceding claims, wherein the formulation further comprises glycerol.

## Patentansprüche

1. Verwendung einer Zusammensetzung, welche ein Allergen und ein aus der aus Sauerstoff enthaltenden Metallsalzen bestehenden Gruppe ausgewähltes Adjuvans aufweist, zur Herstellung einer flüssigen Formulierung zur Vorbeugung oder Behandlung einer Allergie bei einem Subjekt durch oromukosale Verabreichung.

2. Verwendung nach Anspruch 1, wobei das Sauerstoff enthaltende Metallsalz aus der aus Aluminiumhydroxid, Aluminiumphosphat und Calciumphospat bestehenden Gruppe ausgewählt ist.

3. Verwendung nach Anspruch 1, wobei das Sauerstoff enthaltende Metallsalz Aluminiumhydroxid ist.

4. Verwendung nach Anspruch 3, wobei die Konzentration des Sauerstoff enthaltenden Metallsalzes 0,035-1000 mg/ml, bevorzugter 0,10-100 mg/ml, noch bevorzugter 0,25-10 mg/ml und am bevorzugtesten 0,5-5 mg/ml beträgt.

5. Verwendung nach einem der Ansprüche 1 bis 4, wobei die Allergenkonzentration 0,01-100 mg/ml, bevorzugter 0,1-10 mg/ml beträgt.

6. Verwendung nach einem der vorhergehenden Ansprüche, wobei das Allergen aus der aus einem Graspollenallergen, einem Hausstaubmilbenallergen, einem Allergen des Beifußblättrigen Traubenkrauts (Ragweed), einem Zedempollen- oder Katzenallergen, und einem Birkenallergen bestehenden Gruppe ausgewählt ist.

7. Verwendung nach einem der vorhergehenden Ansprüche, wobei die oromukosale Verabreichung eine sublinguale Verabreichung ist.

8. Verwendung nach einem der vorhergehenden Ansprüche, wobei die Formulierung weiters Glycerol aufweist.

## Revendications

1. Utilisation d'une formulation comprenant un allergène et un adjuvant sélectionnés parmi le groupe constitué des sels métalliques contenant de l'oxygène pour la fabrication d'une formulation liquide pour la prévention ou le traitement d'une allergie chez un sujet par administration oromucosale.

2. Utilisation suivant la revendication 1, dans laquelle le sel métallique contenant de l'oxygène est sélectionné parmi le groupe constitué de l'hydroxyde d'aluminium, du phosphate d'aluminium et du phosphate de calcium.

3. Utilisation suivant la revendication 1, dans laquelle le sel métallique contenant de l'oxygène est de l'hydroxyde d'aluminium.

4. Utilisation suivant la revendication 3, dans laquelle la concentration du sel métallique contenant de l'oxygène est 0,035-1000 mg/ml, de manière plus préférée 0,10-100 mg/ml, de manière plus préférée 0,25-10 mg/ml et de manière la plus préférée 0,5-5 mg/ml.

5. Utilisation suivant l'une quelconque des revendications 1-4, dans laquelle la concentration en allergène est 0,01-100 mg/ml, de manière plus préférée 0,1-10 mg/ml.

6. Utilisation suivant l'une quelconque des revendications précédentes, dans laquelle l'allergène est sélectionné parmi le groupe constitué d'un allergène de pollen de plante herbacée, d'un allergène d'acarien détriticole, d'un allergène d'ambroisie, de pollen de cèdre ou d'un allergène félin et d'un allergène de bouleau.

7. Utilisation suivant l'une quelconque des revendications précédentes, dans laquelle l'administration oromucosale est une administration sublinguale.

8. Utilisation suivant l'une quelconque des revendications précédentes, dans laquelle la formulation comprend en outre du glycérol.
